(19) **Europäisches Patentamt / European Patent Office / Office européen des brevets**

(11) **EP 3 140 654 B1**

(12) **FASCICULE DE BREVET EUROPEEN**

(45) Date de publication et mention de la délivrance du brevet:
**02.05.2018 Bulletin 2018/18**

(21) Numéro de dépôt: **15718523.2**

(22) Date de dépôt: **27.03.2015**

(51) Int Cl.:
*G01N 33/573* (2006.01)    *G01N 11/10* (2006.01)
*G01N 33/58* (2006.01)

(86) Numéro de dépôt international:
**PCT/FR2015/050804**

(87) Numéro de publication internationale:
**WO 2015/170021 (12.11.2015 Gazette 2015/45)**

(54) **PROCEDE DE DETERMINATION DE L'ACTIVITE ENZYMATIQUE D'UNE ENZYME ET DISPOSITIF POUR SA MISE EN OEUVRE**

VERFAHREN ZUR BESTIMMUNG DER ENZYMAKTIVITÄT EINES ENZYMS UND VORRICHTUNG ZUR DURCHFÜHRUNG DAVON

METHOD FOR DETERMINING THE ENZYME ACTIVITY OF AN ENZYME AND DEVICE FOR THE IMPLEMENTATION THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.05.2014 FR 1454163**

(43) Date de publication de la demande:
**15.03.2017 Bulletin 2017/11**

(73) Titulaires:
• **Biofilm Control**
**63360 Saint-Beauzire (FR)**
• **Université Blaise Pascal de Clermont-Ferrand**
**F-63000 Clermont-Ferrand (FR)**

(72) Inventeurs:
• **BERNARDI, Thierry**
**F-63170 Perignat-les-sarlieve (FR)**
• **MICHAUD, Philippe**
**F-63160 Billom (FR)**
• **BADEL-BERCHOUX, Stéphanie**
**F-63000 Clermont-ferrand (FR)**
• **PIERRE, Guillaume**
**F-63000 Clermont-ferrand (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**WO-A1-2005/090944**    **WO-A1-2012/001312**

• **BADEL S ET AL: "A new method to screen polysaccharide cleavage enzymes", ENZYME AND MICROBIAL TECHNOLOGY, STONEHAM, MA, US, vol. 48, no. 3, 11 novembre 2010 (2010-11-11), pages 248-252, XP028138958, ISSN: 0141-0229, DOI: 10.1016/J.ENZMICTEC.2010.11.003 [extrait le 2010-11-18]**
• **S. BADEL ET AL: "New Method Showing the Influence of Matrix Components in Leuconostoc mesenteroides Biofilm Formation", APPLIED BIOCHEMISTRY AND BIOTECHNOLOGY, vol. 151, no. 2-3, 10 avril 2008 (2008-04-10), pages 364-370, XP055156649, ISSN: 0273-2289, DOI: 10.1007/s12010-008-8199-y**
• **CHAVANT ET AL: "A new device for rapid evaluation of biofilm formation potential by bacteria", JOURNAL OF MICROBIOLOGICAL METHODS, ELSEVIER, AMSTERDAM, NL, vol. 68, no. 3, 16 février 2007 (2007-02-16), pages 605-612, XP005892101, ISSN: 0167-7012, DOI: 10.1016/J.MIMET.2006.11.010**

## Description

### Domaine technique

La présente invention se rapporte à un procédé de détermination de l'activité enzymatique endo ou exo d'enzymes.

La présente invention se rapporte également à un dispositif de détermination de l'activité endo ou exo d'une enzyme susceptible de mettre en oeuvre le procédé précité.

La présente invention trouve une application notamment dans les domaines analytiques, de la recherche biologique, enzymologique, dans le domaine pharmaceutique et/ou dans le domaine médical.

Dans la description ci-dessous, les références entre crochets ([ ]) renvoient à la liste des références présentée à la fin du texte.

### Etat de la technique

Les enzymes sont des protéines qui sont connues, notamment, pour catalyser des réactions chimiques ou biologiques et ainsi accélérer et/ou faciliter la production de produits sans en modifier la structure. En particulier, les enzymes sont connues pour leur capacité à abaisser l'énergie d'activation d'une réaction chimique et/ou biologique, via, par exemple une mise en place des produits ou composés impliqués dans la réaction dans les meilleures conditions et/ou conformations. Les enzymes peuvent permettre ainsi d'accélérer jusqu'à des millions de fois les réactions chimiques du métabolisme, sans pour autant modifier l'équilibre formé. Les enzymes agissent à faible concentration et elles se retrouvent intactes en fin de réaction.

Il existe de nombreuses enzymes et études concernant l'identification de leurs séquences et la caractérisation de leurs activités. En janvier 2012, il y avait 4 585 enzymes différentes dont la séquence d'acides aminés a pu être répertoriée sur la base de données MACiE du site internet de l'Institut européen de bio-informatique au Royaume-Uni.

Chaque enzyme permet de catalyser, en général un type de réaction enzymatique, par exemple une réaction chimique bien précise, telle que l'hexokinase, qui permet à l'aide d'une molécule d'ATP de phosphoryler le glucose pour obtenir le glucose-6-phosphate.

Par ailleurs, certaines enzymes sont capables de dégrader des biopolymères, selon deux mécanismes endo- ou exo- selon la situation du site de clivage. Le clivage peut être réalisé par exemple au niveau de sites particuliers à l'intérieur du polymère, de la molécule et/ou à partir de ces extrémités. Elles peuvent notamment dégrader des protéines, des polysaccharides, des acides nucléiques etc.

De nombreuses méthodes sont connues afin d'étudier les cinétiques des enzymes, par exemple la méthode de Michaëlis Mentens, permettant de caractériser la vitesse de catalyse de la réaction par l'enzyme, ses propriétés d'affinités pour son substrat etc.

Toutefois, ces méthodes ne permettent pas de distinguer, par exemple dans le cas où l'enzyme est une nucléase, s'il s'agit d'une endo ou d'une exonucléase.

Par ailleurs, les méthodes de l'état de la technique nécessitent une étude au cas par cas qui doit être adaptée à chaque enzyme et qui ne sont pas adaptables pour une analyse en haut débit.

En outre, les méthodes de l'état de la technique, de par la non possibilité d'automatisation, nécessitent la présence de personnes hautement qualifiées et sont des procédés longs et coûteux.

Il existe donc un réel besoin de trouver un procédé palliant ces défauts, inconvénients et obstacles de l'art antérieur, en particulier d'un procédé permettant de détecter et/ou discriminer les activités enzymatiques, un procédé utilisable en haut débit, de réduire les coûts pour sa mise en oeuvre et l'obtention des résultats associés.

### Description de l'invention

La présente invention permet de résoudre les problèmes et inconvénients des procédés de l'état de la technique en fournissant un procédé de détermination de l'activité endo ou exo d'une enzyme test comprenant les étapes suivantes:

a) Introduire dans un milieu liquide au moins une particule magnétique ou magnétisable ou chargée électriquement, ladite particule flottant à la surface du milieu liquide ou en suspension dans le milieu liquide ou reposant sur une surface immergée dans le milieu liquide;

b) Introduire dans ledit milieu un substrat de ladite enzyme test à une concentration déterminée [S];

c) Introduire dans ledit milieu comprenant ledit substrat ladite enzyme test,

d) Mesurer un premier degré de liberté (DL) de mouvement de ladite au moins une particule dans ledit milieu à un temps $t_0$ correspondant à l'introduction de ladite enzyme dans ledit milieu,

e) Appliquer un champ magnétique, électrique ou électromagnétique capable de mettre en mouvement ladite au moins une particule flottant à la surface du milieu liquide ou en suspension dans le milieu liquide ou reposant sur une surface immergée dans le milieu liquide pendant un temps déterminé;

f) Mesurer au moins un second degré de liberté (DL) de mouvement de ladite au moins une particule dans ledit milieu à un second temps $t_{>0}$,

g) Calculer la variation de degré de liberté (DL), $\Delta DL$ entre le second temps $t_{>0}$ par rapport au temps $t_0$

h) Répéter les étapes a) à g) avec différentes concentrations dudit substrat ;

i) A partir d'un graphique obtenu avec les valeurs mesurées en faisant apparaitre en abscisses les valeurs de temps et en ordonnées les valeurs de $\Delta DL$, déterminer la vitesse enzymatique initiale ($v_i$) en $\Delta DL$ par unité de temps pour différentes concentrations en substrat, correspondant à la tangente à l'origine dudit graphique,

j) Déterminer la constante $K_{ERT}$ correspondant à la capacité de l'enzyme à modifier le degré de liberté de mouvement de ladite particule dans ledit milieu, à partir de la courbe obtenue avec les valeurs mesurées en faisant apparaitre en abscisse 1/vi et en ordonnée [S]/vi correspondant à la formule suivante :

$$\frac{1}{V_i} = a \times \frac{[S]}{V_i} + b$$

dans laquelle a est la pente de la courbe obtenue et b est l'ordonnée à l'origine, la valeur de $K_{ERT}$ est égale à la valeur absolue de l'intersection de la courbe avec l'abscisse,

k) Comparer la valeur de la constante $K_{ERT}$ obtenue à l'étape i) avec une constante $K_{ERT}$ de référence obtenue à partir d'une enzyme de référence dont l'activité enzymatique est préalablement connue pour ledit substrat, ladite comparaison permettant de déterminer l'activité endo ou exo de ladite enzyme.

Les inventeurs ont démontré de manière surprenante que le procédé de l'invention, de par la détermination de la valeur $K_{ERT}$, permet de détecter l'activité enzymatique, et ainsi établir si ladite enzyme test est une endo et/ou une exo. En effet, de manière surprenante, les inventeurs de la présente invention ont démontré que les valeurs de $K_{ERT}$ diffèrent en fonction du mécanisme exo- ou endo- des enzymes. En particulier, les inventeurs de la présente invention ont montré de manière surprenante que le procédé de l'invention permet avantageusement, pour une même activité catalytique de discriminer un mécanisme endo- et/ou exo.

Les inventeurs ont également démontré de manière surprenante que le procédé selon l'invention permet de différencier deux enzymes tests selon leur(s) mécanisme(s) d'action(s), par exemple différencier une endo-enzyme d'une exo-enzyme.

Avantageusement, les inventeurs ont également démontré de manière surprenante que le procédé selon l'invention permet pour une même enzyme test de déterminer le type d'interaction, par exemple une liaison covalente, une liaison ionique, intervenant entre l'enzyme test et ledit substrat.

Dans la présente « ERT » signifie « Enzyme Ring Test ».

Dans la présente par « l'activité enzymatique » on entend tout type de réaction catalysée par une enzyme. Il peut s'agir par exemple d'une réaction de clivage, de synthèse, par exemple de polymères, susceptibles d'être catalysés par une enzyme.

Dans la présente par « enzyme » on entend toute enzyme connue de l'homme du métier. Il peut s'agir par exemple d'une enzyme bactérienne, de mammifère, virale, fongique, végétale. Il peut s'agir par exemple d'enzymes catalysant des réactions de polymérisation, des réactions de dépolymérisation, des réactions de clivage, par exemple de polymères, par exemple de polysaccharides, d'acides nucléiques, protéines, polyhydroxyalcanoates, polyesters, polythioesters, polyesters inorganiques, polyisoprénoïdes

Selon l'invention, l'enzyme test peut être présente dans une solution, par exemple une solution biologique, par exemple du sang, de la lymphe, de l'urine et/ou toute solution biologique connue de l'homme du métier.

Selon l'invention, l'enzyme test peut être présente dans tout extrait protéique, par exemple un extrait de plantes, de cultures bactériennes ou fongiques et/ou tout extrait protéique connue de l'homme du métier.

Il peut s'agir de tout extrait protéique obtenu par tout procédé connu de l'homme du métier, il peut s'agir par exemple d'un extrait brut, fractionné, par exemple par centrifugation, par filtration et/ou purifié, par exemple par chromatographie et/ou tout moyen et/ou procédé connu de l'homme du métier

Avantageusement, selon l'invention lorsque l'enzyme test est présente dans une solution et/ou extrait, le procédé de l'invention permet avantageusement d'identifier et/ou de quantifier l'activité enzymatique de ladite enzyme test.

Avantageusement, selon l'invention lorsque l'enzyme test est une enzyme dépolymérisante, notamment dans une solution et/ou extrait tel que défini ci-dessus, le procédé de l'invention permet avantageusement de déterminer le mécanisme enzymatique, notamment l'endo ou exo de ladite enzyme test, par exemple par comparaison de ses caractéristiques cinétiques avec des valeurs de références obtenues avec une enzyme de référence.

Avantageusement, l'activité exo enzyme ou endo de enzyme l'enzyme de référence est connue et on compare les valeurs de $K_{ERT}$ obtenues pour l'enzyme test à l'étape j) avec les valeurs de $K_{ERT}$ obtenues avec l'enzyme de référence. La comparaison des valeurs de $K_{ERT}$ permet avantageusement de déterminer le mécanisme enzymatique, notamment l'endo ou exo de ladite enzyme test.

Selon l'invention, lorsque l'enzyme test est présente dans une solution et/ou extrait, le procédé selon l'invention peut comprendre une étape préalable de détection de l'activité [E] de ladite enzyme test dans ladite solution et/ou ledit extrait

Selon l'invention, lorsque l'enzyme test est présente dans une solution, ladite solution peut correspondre au milieu liquide tel que défini ci-dessous.

Avantageusement, lorsque la solution et/ou extrait comprend l'enzyme test tel que décrit ci-dessus, le procédé selon l'invention ne comprend pas l'étape c), et les étapes a) et b) d'introduction d'au moins une particule et d'un substrat peuvent être réalisées simultanément dans ladite solution et/ou extrait.

Dans la présente par « substrat » on entend toute molécule connue de l'homme du métier susceptible de subir l'action d'une enzyme telle que définie ci-dessus. Il peut s'agir par exemple de substrats issus de cellules eucaryotes, de cellules procaryotes, de virus. Il peut s'agir par exemple de polymères, par exemple de polysaccharides, d'acides nucléiques, protéines, polyhydroxyalcanoates, polyesters, polythioesters, polyesters inorganiques, polyisoprénoïdes polysaccharides d'origines végétales, animales.

Dans la présente par degré de liberté (DL), on entend la possibilité de mouvement dans l'espace de ladite au moins une particule dans ledit milieu.

Selon l'invention la mesure du degré de liberté de mouvement de ladite au moins une particule peut être effectuée par tout procédé connu de l'homme du métier. Il peut s'agir par exemple d'un procédé de détection optique, par exemple utilisant une caméra, un microscope optique, appareil photo, d'un procédé de détection optique n'utilisant pas de microscope à balayage.

Avantageusement, lorsque la mesure du degré de liberté de mouvement de ladite au moins une particule est effectuée avec un procédé de détection optique, une mesure des composantes rouge, bleu et/ou verte des pixels contenus dans l'image peut être effectuée.

Selon l'invention, la mesure du degré de liberté de mouvement de ladite au moins une particule peut être effectuée sur une surface qui peut représenter une surface d'observation, par exemple sur une première image, par exemple sur une surface de 1 à 10 000, de 10 à 900 de 50 à 700, de 100 à 500 pixels.

Selon la présente invention la taille d'un pixel est définie par sa largeur $\times$ hauteur, par exemple la hauteur 0,018 à 0,660 mm, la largeur de 0,018 à 0,660 mm

Selon l'invention la mesure du degré de liberté de mouvement de ladite au moins une particule peut être effectuée sur une surface qui peut représenter une surface d'observation par exemple sur une zone circulaire, une zone elliptique. Il peut s'agir par exemple d'une zone circulaire centrée, par exemple sur l'axe de magnétisation et de rayon 2,25mm.

Selon l'invention, une valeur minimale du degré de liberté correspond à une absence totale de liberté de mouvement de ladite au moins une particule. Une valeur maximale du degré de liberté correspondant à une liberté de mouvement totale de ladite au moins une particule.

Selon l'invention, le degré de liberté de mouvement de ladite au moins une particule peut être fonction de la densité du milieu et/ou de la concentration dudit substrat présent dans ledit milieu. En d'autres termes, selon l'invention, le degré de mouvement de ladite au moins une particule est fonction de la concentration et/ou quantité dudit substrat présent dans ledit milieu. Plus la concentration et/ou quantité dudit substrat est importante, plus le degré de liberté de ladite au moins une particule est faible. Plus la concentration et/ou quantité dudit substrat est faible, plus le degré de liberté de ladite au moins une particule est important. Aussi, selon l'invention, le degré de liberté de ladite au moins une particule peut être fonction de l'activité enzymatique de l'enzyme test.

Selon l'invention, la variation de degré de liberté peut être calculée selon les procédés connus de l'homme du métier pour calculer une variation entre deux mesures utilisant un procédé de détection optique. Il peut s'agir par exemple d'un procédé tel que décrit dans le document Chavant et al 2007 [4].

Selon l'invention, la détermination de la vitesse initiale (vi) en ΔDL par unité de temps peut être effectuée par mesure du degré de liberté de ladite au moins une particule en fonction de la concentration de substrat présente dans le milieu en présence de ladite enzyme test et en fonction du temps, la vitesse initiale étant calculée selon la formule suivante :

$$Vi = \Delta DL/temps$$

Selon l'invention, la variation du degré de liberté peut être corrélée à une valeur particulière dénommée index Enzyme (EI) correspondant à l'écart type des moyennes des mesures de ΔDL. L'index Enzyme (EI) correspond à l'index Biofilm (BFI) décrit dans le document Chavant et al 2007 [4]. Il peut être égal par exemple à l'écart type des moyennes des composantes rouge, bleu et verte des pixels contenus dans l'image obtenue après magnétisation à laquelle l'image obtenue avant magnétisation a été retranchée. La mesure des composantes rouge, bleu et verte peut être réalisée, par exemple sur une surface d'observation telle que mentionnée ci-dessus, par exemple sur une zone circulaire centrée, par exemple sur l'axe de magnétisation et de rayon 2,25 mm.

Selon l'invention, l'index Enzyme (EI) peut correspondre à la valeur de l'index Biofilm (BFI). Aussi, le procédé de l'invention peut être également mis en oeuvre en remplaçant la mesure du degré de liberté (DL) par la mesure de BFI ou EI et la détermination de la vitesse initiale vi et de la valeur $K_{ERT}$ en BFI/min ou $g.min.BFI^{-1}.L^{-1}$ correspondant également à la détermination de la vitesse initiale vi et de la valeur $K_{ERT}$ en EI/min ou g.min.EI-1.L-1.

Selon l'invention, la mesure de l'index Enzyme et/ou de l'index Biofilm peut être calculée par un logiciel, par exemple le logiciel BFC Elements EZ (marque déposée).

Selon l'invention, le milieu liquide peut être tout milieu liquide connu de l'homme du métier adapté pour la mise en oeuvre du procédé et/ou avec l'enzyme test. Il peut s'agir par exemple d'un milieu tampon, par exemple tout milieu tampon connu de l'homme du métier, par exemple un milieu tampon disponible dans le commerce, par exemple du tampon phosphate salin (PBS).

Selon l'invention, ladite au moins une particule, peut être choisie dans le groupe comprenant une particule chargée électriquement, une particule magnétique, une particule revêtue d'au moins une couche magnétique, une particule magnétisable, une particule revêtue d'une couche magnétisable, une particule électrique, électromagnétique, électrisable, portant une charge électrique ou un mélange de deux ou plusieurs de ces particules. En fait, il peut s'agir de toute particule permettant de mettre en oeuvre la présente invention.

Avantageusement, ladite au moins une particule peut être une particule de toute forme adaptée à la mise en oeuvre de la présente invention, par exemple sous forme de bille, de palet, de forme géométrique asymétrique, par exemple avec une face plane, etc.

Toute taille appropriée de particule magnétique peut être utilisée. La taille peut être choisie par exemple en fonction de la taille du contenant de la solution. Par exemple, la taille des particules peut être inférieure au dixième de la taille du contenant, de préférence inférieure au centième, de manière encore plus préférée inférieure au millième de la taille du contenant. Par exemple la particule peut présenter une taille de, par exemple, 10 nm à 100 nm, de 0,1 à 100 $\mu$m.

Selon l'invention, le procédé de l'invention peut être mis en oeuvre avec une pluralité de particules, par exemple avec au moins 2 particules, avec par exemple de 2 à 10 000 000, de 1000 à 1 000 000, de de 10 000 à 1 000 000, de 100 000 à 1 000 000, de 10 000 à 100 000.

La pluralité de particules permet avantageusement de mesurer directement, sans dispositif complexe de visualisation et sans colorant, le degré de liberté de mouvement desdites particules. Selon l'invention, lorsque le procédé est mis en oeuvre avec une pluralité de particules, lesdites particules peuvent être de taille identique ou différente.

Lorsque les particules sont de tailles différentes, les petites particules peuvent présenter une taille, par exemple, de 10 nm à 1 $\mu$m, par exemple de 100 à 500nm, et les grosses particules peuvent présenter une taille, par exemple, de 1 $\mu$m à 100$\mu$m, par exemple de 1 $\mu$m à 10 $\mu$m, par exemple de 1 $\mu$m à 5 $\mu$m.

Selon l'invention, on peut également utiliser une pluralité de particules de tailles identiques avec une magnétisation différente.

Selon l'invention, ladite, au moins une, particule est de préférence une particule génératrice d'un signal détectable. La détection de ce signal sera fonction des propriétés de la particule. À titre d'exemple, ladite, au moins une, particule peut être fluorescente, phosphorescente, radioactive, chimioluminescente, réfléchissante ou colorée.

Par exemple dans le cas où ladite, au moins une, particule est fluorescente, la fluorescence émise par la particule peut être détectée par exemple visuellement, et/ou par tous moyens optiques connus de l'homme du métier. Ladite, au moins une, particule peut être par exemple éclairée, pour suivre son mouvement au moyen d'une source lumineuse, par exemple par un faisceau laser.

Par exemple dans le cas où la, au moins une, particule est phosphorescente, cette particule peut être visualisée par exemple visuellement, par tous moyens optiques connus de l'homme du métier.

Par exemple dans le cas où la particule est radioactive, cette particule peut être détectée même au travers de liquides ou milieux de cultures optiquement opaques, ainsi qu'au travers de plaques de microdilution optiquement opaques par tous dispositifs de détection de la radioactivité émise connue de l'homme du métier, notamment la méthode classique de révélation sur film autoradiographique. Il suffit alors de plaquer un film sensible sous le contenant comprenant ledit milieu liquide et de révéler ensuite l'image. Par exemple dans le cas où là, au moins une, particule est chimioluminescente la détection de la particule peut se former par ajout dans le milieu du réactif chimique permettant l'émission d'énergie lumineuse par les particules. La détection de ce signal peut être par exemple visuelle et/ou par tous moyens connus de l'homme du métier notamment par l'utilisation de caméra CCD (« Charges Coupled Device ») sensible aux longueurs d'ondes émises, qui balaient (scannent) les cupules de la plaque de microdilution.

Par exemple dans le cas où la, au moins une, particule est réfléchissante la détection de la particule peut être par exemple visuelle ou par tous moyens optiques connus de l'homme du métier. Avantageusement, ladite, au moins une, particule peut être par exemple éclairée, pour suivre son mouvement au moyen d'une source lumineuse, par exemple par un faisceau laser.

L'homme du métier comprendra aisément que pour la réalisation de la présente invention, le choix des propriétés visuelles desdites, au moins deux, particules peut également se faire en fonction du milieu liquide. En effet la détection du mouvement desdites, au moins deux, particules est d'autant plus facile que le contraste entre lesdites, au moins deux, particules et le milieu liquide est grand.

Dans la présente invention, le champ magnétique, électrique ou électromagnétique peut être tout champ permettant de mettre en mouvement ladite, au moins une particule, par exemple un champ électromagnétique ou un champ magnétique. Le champ magnétique, ou électrique ou électromagnétique peut être généré, par exemple par un aimant ou par un solénoïde. L'aimant peut être par exemple sous forme de barreau, de pointe, de pièce, etc. ou toute forme appropriée pour la mise en oeuvre de la présente invention. Le champ peut, par exemple être appliqué par tout moyen connu de l'homme du métier, par exemple par impulsion, par augmentation progressive du champ électromagnétique, par variations de champ électromagnétique ou par une combinaison ces applications.

Une augmentation progressive du champ électromagnétique peut être obtenue, par exemple, par rapprochement d'un aimant selon une trajectoire rectiligne ou sinusoïdale, ou selon un mouvement oscillant présentant ou non une amplitude d'oscillation et/ou une fréquence variables. Des variations de champ plus complexes peuvent être obtenues, par exemple par rotation ou par des combinaisons de mouvements d'un matériau aimanté à proximité de ladite, au moins une, particule.

Ainsi, selon l'invention, ledit champ magnétique peut être généré par des moyens générateurs de champ qui peuvent être ou non en mouvement.

Lorsque plusieurs particules doivent être mises en mouvement, le champ doit pouvoir regrouper lesdites particules sur ladite surface immergée dans ledit milieu liquide.

Quel que soit le mode de mise en oeuvre de l'invention, le procédé de l'invention peut avantageusement être réalisé simultanément dans une pluralité de compartiments. Dans la présente, par compartiment dans la présente invention, on entend par exemple un réacteur de culture, des cupules, des tubes ou des puits par exemple de plaques de microdilution. On entend par plaque de microdilution le type de plaque défini par exemple par l'American National Standards Institute et la Society for Biomolecular Screening (« microplates ») portant 96 puits, 384 et même 1536.

Dans ce cas, pour la mise en mouvement des particules dans lesdits compartiments, une pluralité de champs magnétiques, par exemple d'aimants, peuvent avantageusement être utilisés. Les aimants peuvent par exemple être fixés sur un support de telle manière à permettre une juxtaposition de chaque compartiment dans lequel on réalise le procédé de l'invention avec un aimant du support. L'application du champ magnétique sur lesdits compartiments est indépendante d'un compartiment à un autre. Le champ magnétique appliqué auxdits compartiments peut être identique ou différent d'un compartiment à l'autre, de préférence identique.

Selon l'invention, les différents compartiments peuvent être regroupés sur un même support, par exemple sur une plaque comportant de 1 à 1536 puits, par exemple 6, 16, 64, 96, etc.

Le compartiment peut correspondre par exemple à une enceinte avec une extrémité fermée, du type tube, puits, etc. ou une enceinte présentant deux ouvertures.

Selon une première configuration, le compartiment peut présenter une extrémité fermée de sorte à former un fond plat.

Selon une seconde configuration, le compartiment peut présenter une extrémité fermée de sorte à former un fond hémisphérique.

Selon l'invention, le champ magnétique, électromagnétique, électrique peut être appliqué, par exemple pendant un temps de 1 seconde à 15 minutes, de 10 secondes à 10 minutes.

Bien entendu, l'homme du métier de par ses connaissances générales saura adapter le temps en fonction des enzymes tests et/ou de la puissance du champ appliqué.

La présente invention a également pour objet un dispositif de détermination de l'activité enzymatique d'une enzyme comprenant la mise en oeuvre du procédé selon l'invention.

En particulier, la présente invention a également pour objet un dispositif comprenant:

- au moins un compartiment destiné à recevoir ledit milieu liquide comprenant ladite au moins une particule et ladite enzyme test.
- des moyens pour générer un champ électrique, magnétique ou électromagnétique, de préférence un champ magnétique, ledit champ étant appliqué à ladite particule,
- des moyens d'observation du degré de liberté de ladite au moins une particule,
- des moyens de détermination de ladite vitesse initiale enzymatique, et
- des moyens de détermination de ladite constante $K_{ERT}$ correspondant à la capacité de l'enzyme à modifier le degré de liberté de mouvement de ladite particule dans ledit milieu en présence dudit substrat.

Selon l'invention, le compartiment est tel que définie ci-dessus.

Selon l'invention, ladite particule est telle que définie ci-dessus.

Selon l'invention, les moyens pour générer un champ électrique, magnétique ou électromagnétique sont tels que définis ci-dessus.

Selon l'invention, les moyens d'observation du degré de liberté sont tels que définis ci-dessus.

Selon l'invention, les moyens de détermination de la vitesse initiale et/ou de de la constante $K_{ERT}$ peuvent être par exemple un logiciel informatique mis en oeuvre par un ordinateur. Il peut s'agir par exemple d'un logiciel de calcul comprenant l'acquisition des valeurs et le calcul de la vitesse initiale et/ou de la constante $K_{ERT}$.

D'autres avantages pourront encore apparaître à l'homme du métier à la lecture des exemples ci-dessous, illustrés par les figures annexées, données à titre illustratif.


Brève description des figures


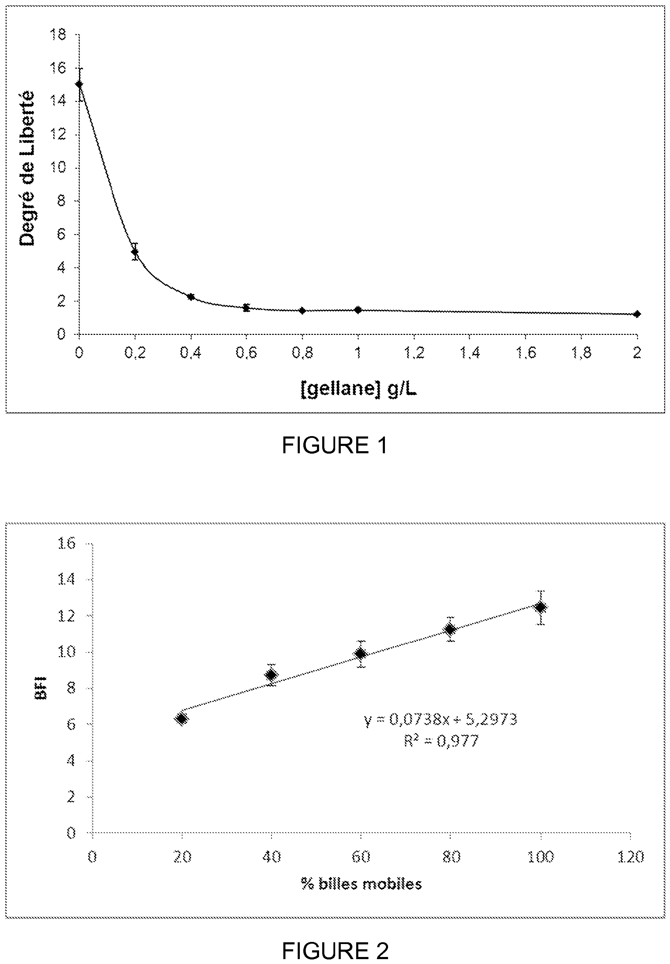

La figure 1 représente un diagramme mesurant le degré de liberté (abscisse) en fonction de la concentration de gellane en g/L dans une solution.

La figure 2 représente un diagramme mesurant l'index biofilm (BFI) (ordonnée) en fonction du pourcentage de particules (billes) mobiles dans le milieu

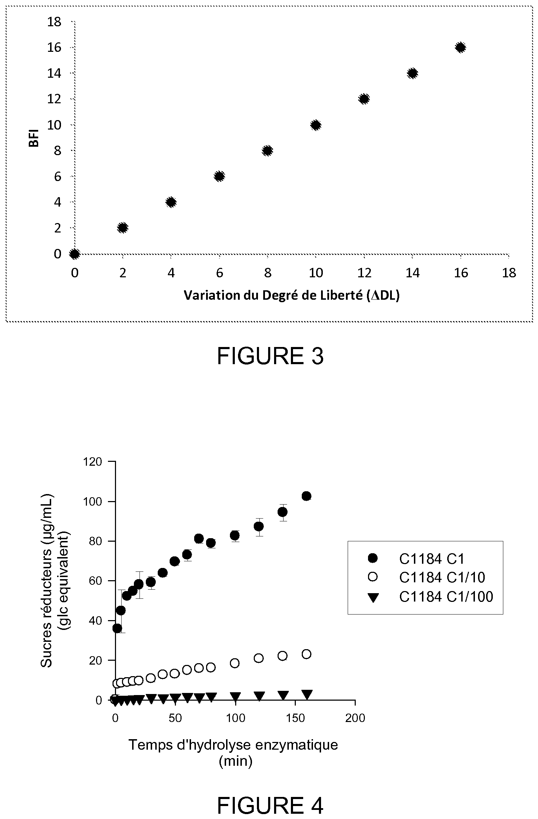

La figure 3 représente un diagramme mesurant l'index biofilm (BFI) (abscisse) en fonction de la variation degré de liberté de particules (billes) mobiles dans le milieu. La figure 4 représente un diagramme représentant la concentration de sucres réducteurs en $\mu$g/ml en fonction du temps en minutes en fonction de l'hydrolyse enzymatique de la CarboxyMéthylCellulose (3 g/L) par l'endocellulase C1184. Sur la figure, les ronds pleins représentent la mesure lorsque le milieu comprend une concentration de C1184 à une concentration de $1,2 \times 10^{-1}$ U/mL, les ronds vides représentent la mesure lorsque le milieu comprend une concentration de C1184 égale à C1/10: $1,2.10^{-2}$ U/mL les triangles pleins représentent la mesure lorsque le milieu comprend une concentration de C1184 égale à C1/100 ($1,2.10^{-3}$ U/mL).

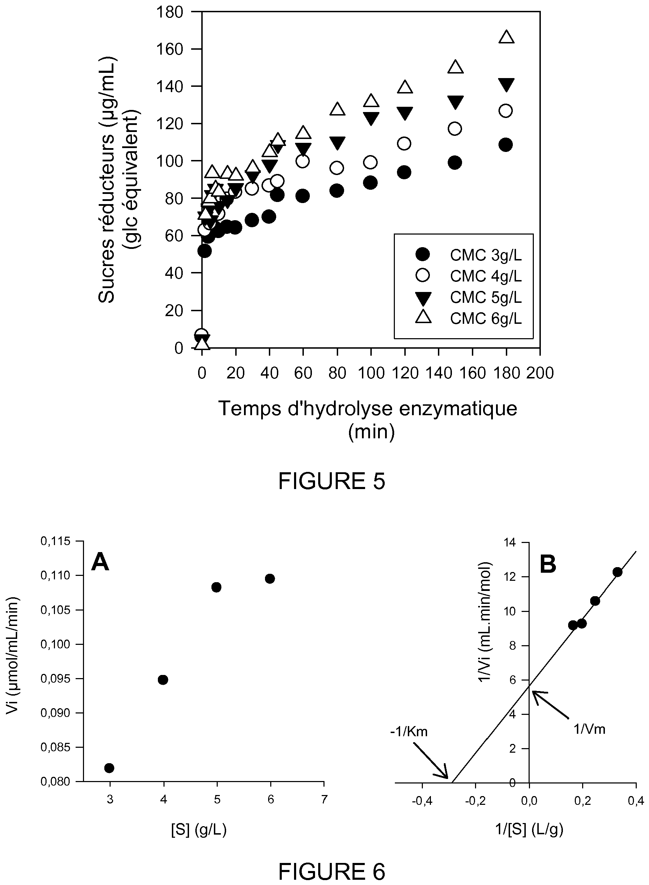

La figure 5 représente un diagramme représentant la concentration de sucres réducteurs en $\mu$g/ml en fonction du temps en minutes en fonction de l'hydrolyse enzymatique de la CMC à différentes concentrations (3 à 6 g/L) par l'endocellulase C1184 à la concentration de $1,2.10^{-2}$ U/mL. Sur la figure, les ronds pleins représentent la mesure lorsque le milieu comprend une concentration de CMC égale à 3g/L, les ronds vides représentent la mesure lorsque le milieu comprend une concentration de CMC égale à 4g/L, les triangles pleins représentent la mesure lorsque le milieu comprend une concentration de CMC égale à 5 g/L, les triangles vides représentent la mesure lorsque le milieu comprend une concentration de CMC égale à 6g/L.

La figure 6 représente une courbe de saturation (figure 6A) et représentation de Lineweaver-Burk (figure 6 B) pour l'endocellulase C1184 à une concentration de $1,2.10^{-2}$ U/mL en présence de CMC à des concentrations variables de 3 à 6 g/L.

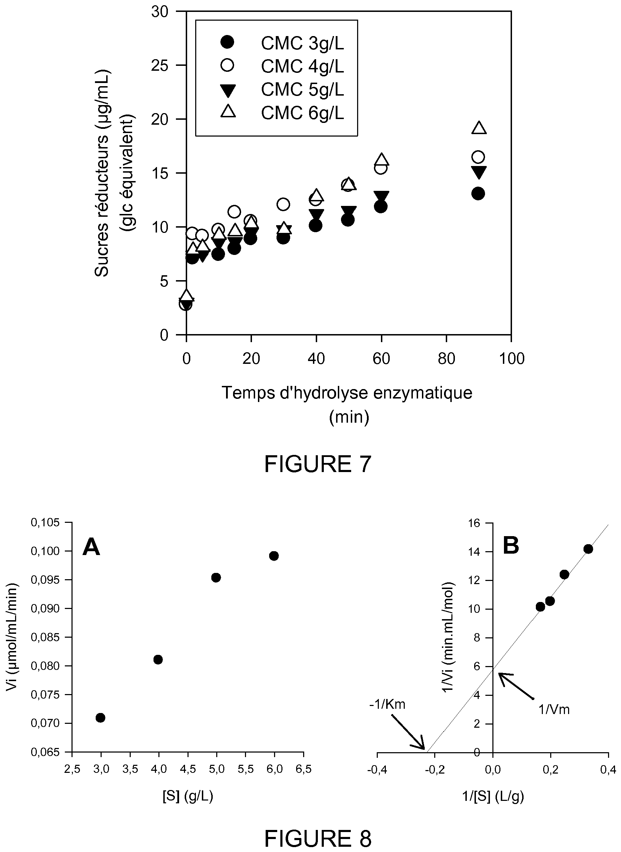

La figure 7 représente l'hydrolyse enzymatique de la CMC à différentes concentrations de 3 à 6 g/L par l'exocellulase CBH1 à la concentration de $5.10^{-4}$ U/mL. Sur la figure, les ronds pleins représentent la mesure lorsque le milieu comprend une concentration de CMC égale à 3g/L, les ronds vides représentent la mesure lorsque le milieu comprend une concentration de CMC égale à 4g/L, les triangles pleins représentent la mesure lorsque le milieu comprend une concentration de CMC égale à 5g/L, les triangles vides représentent la mesure lorsque le milieu comprend une concentration de CMC égale à 6g/L.

La figure 8 représente une courbe de saturation Vi en fonction de la concentration en substrat (figure 8 A) et représentation de Lineweaver-Burk (figure 8 B) pour l'exocellulase CBH1 à une concentration de $5.10^{-4}$ U/mL en présence de CMC à concentrations variables de 3 à 6 g/L.

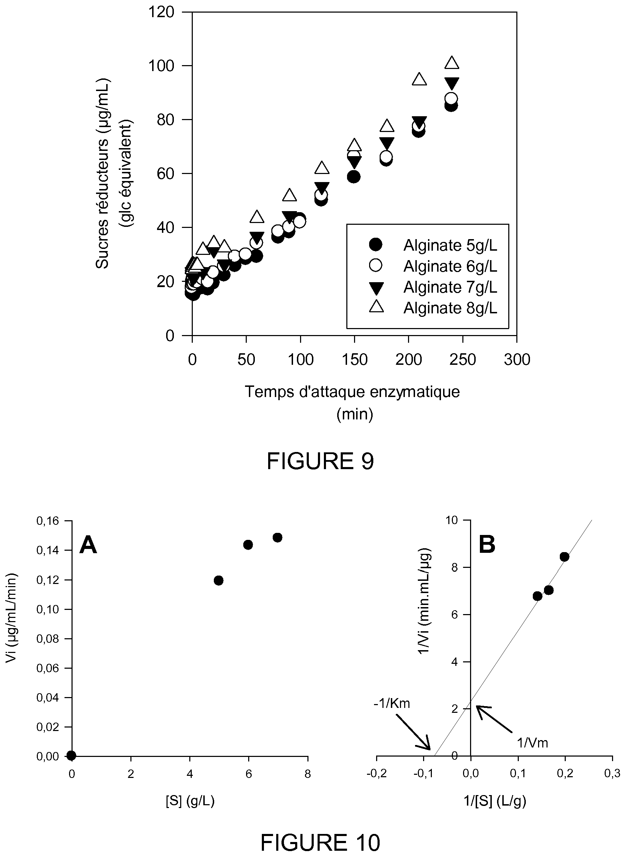

La figure 9 représente les courbes obtenues par béta élimination enzymatique de l'alginate à différentes concentrations de 5 à 8 g/L par l'alginate lyase E-ALGS à une concentration égale à $2.10^{-2}$ U/mL. Sur la figure, les ronds pleins représentent la mesure lorsque le milieu comprend une concentration d'alginate égale à 5g/L, les ronds vides représentent la mesure lorsque le milieu comprend une concentration d'alginate égale à 6g/L, les triangles pleins représentent la mesure lorsque le milieu comprend une concentration d'alginate égale à 7g/L, les triangles vides représentent la mesure lorsque le milieu comprend une concentration d'alginate égale à 8 g/L.

La figure 10 représente une courbe de saturation Vi en fonction de la concentration en substrat (figure 10 A) et représentation de Lineweaver-Burk (figure 10 B) pour l'alginate lyase E-ALGS à une concentration de $2.10^{-2}$ U/mL en présence d'alginate à concentrations variables de 0 à 7 g/L.

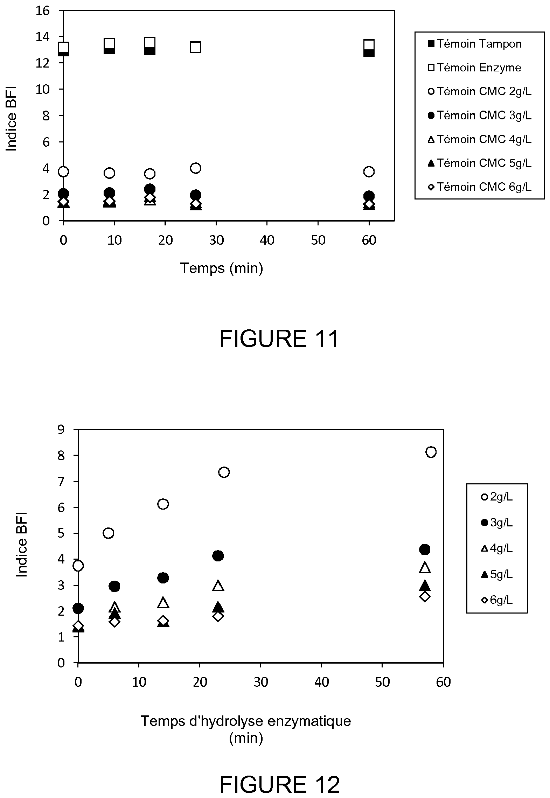

La figure 11 représente une courbe de l'évolution des indices BFI au cours du temps des témoins tampon, tampon et endocellulase C1184 (0,177 U/mL) et tampon et CMC à différentes concentrations. Sur la figure, les carrés pleins représentent les mesures dans le milieu tampon, les carrés vides représentent les mesures dans le milieu comprenant uniquement l'enzyme, les ronds vides représentent les mesures dans le milieu comprenant une concentration de CMC égale à 2g/L, les ronds pleins représentent la mesure lorsque le milieu comprend une concentration de CMC égale à 3g/L, les triangles pleins représentent la mesure lorsque le milieu comprend une concentration de CMC égale à 5 g/L, les triangles vides représentent la mesure lorsque le milieu comprend une concentration de CMC égale à 4 g/L, les losanges vides représentent la mesure lorsque le milieu comprend une concentration de CMC égale à 6 g/L

La figure 12 représente l'évolution des indices BFI mesurés au cours du temps lors de l'hydrolyse enzymatique par l'endocellulase C1184 (0,177 U/mL) de CMC à différentes concentrations de 2 à 6 g/L. Sur la figure, les ronds vides représentent les mesures dans le milieu comprenant une concentration de CMC égale à 2g/L, les ronds pleins représentent la mesure lorsque le milieu comprend une concentration de CMC égale à 3g/L, les triangles pleins représentent la mesure lorsque le milieu comprend une concentration de CMC égale à 5 g/L, les triangles vides représentent la mesure lorsque le milieu comprend une concentration de CMC égale à 4 g/L, les losanges vides représentent la mesure lorsque le milieu comprend une concentration de CMC égale à 6 g/L.

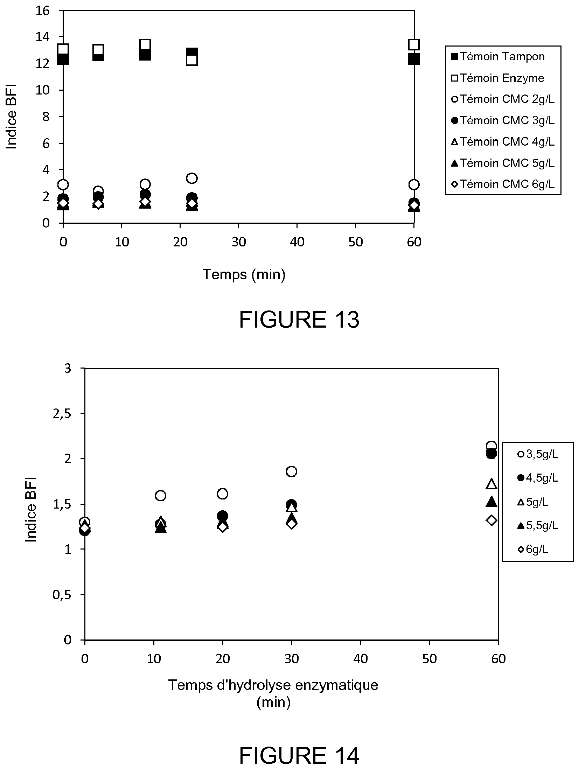

La figure 13 représente des courbes représentant l'évolution des indices BFI mesuré au cours du temps des témoins tampon, tampon et exocellulase CBH1 (0,174 U/mL) et tampon + CMC à différentes concentrations. Sur la figure, les carrés pleins représentent les mesures dans le milieu tampon, les carrés vides représentent les mesures dans le milieu comprenant uniquement l'enzyme, les ronds vides représentent les mesures dans le milieu comprenant une concentration de CMC égale à 2g/L, les ronds pleins représentent la mesure lorsque le milieu comprend une concentration de CMC égale à 3g/L, les triangles pleins représentent la mesure lorsque le milieu comprend une concentration de CMC égale à 5 g/L, les triangles vides représentent la mesure lorsque le milieu comprend une concentration de CMC égale à 4 g/L, les losanges vides représentent la mesure lorsque le milieu comprend une concentration de CMC égale à 6 g/L.

La figure 14 représente des courbes représentant l'évolution des indices BFI mesurés au cours du temps lors de l'hydrolyse enzymatique par l'exocellulase CBH1 (0,174 U/mL) de CMC à différentes concentrations de 2 à 6 g/L. Sur la figure, les ronds vides représentent les mesures dans le milieu comprenant une concentration de CMC égale à 3,5 g/L, les ronds pleins représentent la mesure lorsque le milieu comprend une concentration de CMC égale à 4,5 g/L, les triangles pleins représentent la mesure lorsque le milieu comprend une concentration de CMC égale à 5,5 g/L, les triangles vides représentent la mesure lorsque le milieu comprend une concentration de CMC égale à 5 g/L, les losanges vides représentent la mesure lorsque le milieu comprend une concentration de CMC égale à 6 g/L.

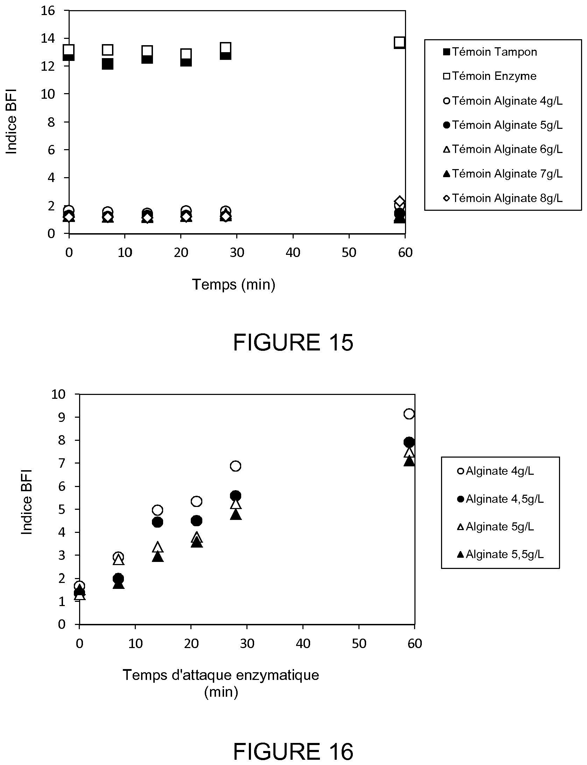

La figure 15 représente des courbes représentant l'évolution des indices BFI mesurés au cours du temps des témoins tampon, tampon et alginate lyase E-ALGS (2,4.10$^{-3}$ U/mL) et tampon et alginate à différentes concentrations. Sur la figure, les carrés pleins représentent les mesures dans le milieu tampon, les carrés vides représentent les mesures dans le milieu comprenant uniquement l'enzyme, les ronds pleins représentent la mesure lorsque le milieu comprend une concentration d'alginate égale à 5g/L, les ronds vides représentent la mesure lorsque le milieu comprend une concentration d'alginate égale à 4g/L, les triangles pleins représentent la mesure lorsque le milieu comprend une concentration d'alginate égale à 7g/L, les triangles vides représentent la mesure lorsque le milieu comprend une concentration d'alginate égale à 6 g/L, les losanges vides représentent la mesure lorsque le milieu comprend une concentration d'alginate égale à 8 g/L.

La figure 16 représente des courbes représentant l'évolution des indices BFI mesurés au cours du temps lors de l'attaque enzymatique par l'alginate lyase E-ALGS (2,4.10$^{-3}$ U/mL) d'alginate à des concentrations allant de 4 à 5,5 g/L. Sur la figure, les ronds pleins représentent la mesure lorsque le milieu comprend une concentration d'alginate égale à 4,5g/L, les ronds vides représentent la mesure lorsque le milieu comprend une concentration d'alginate égale à 4g/L, les triangles pleins représentent la mesure lorsque le milieu comprend une concentration d'alginate égale à 5,5g/L, les triangles vides représentent la mesure lorsque le milieu comprend une concentration d'alginate égale à 5 g/L.

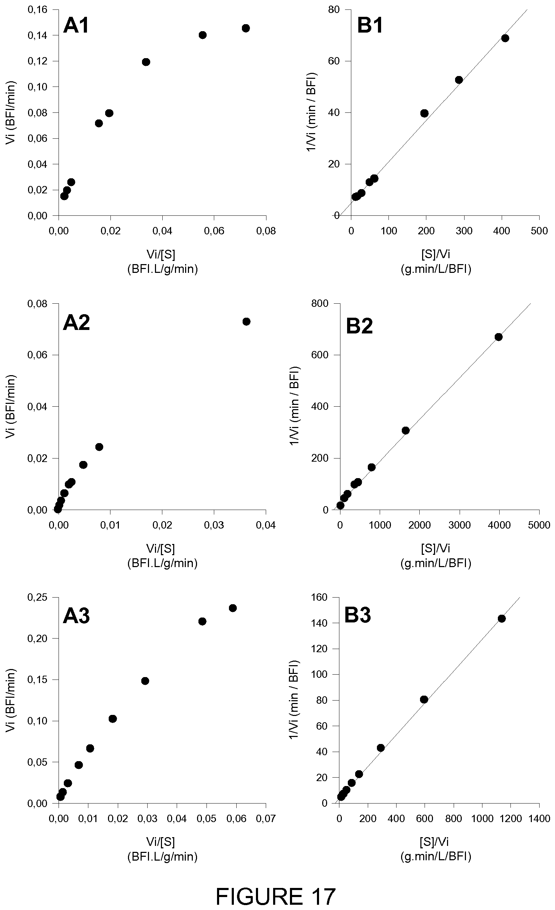

La figure 17 représente les courbes obtenues (figures 17 A1 à B3) après application du procédé et de la formule pour le traitement des données obtenues par (figures 17 A) Vi = f(Vi/[S]) et (figures 17B) 1/Vi = f([S]/Vi) pour les couples (1) CMC/endocellulase C1184, (2) CMC/exocellulase CBH1 et (3) alginate/alginate lyase E-ALGS. Sur ces figures 17 B1 à B3 représente 1/vi et l'abscisse [S]/vi, et sur les figures 17 A1 à A3 l'ordonnée représente la vi et l'abscisse vi/[S].

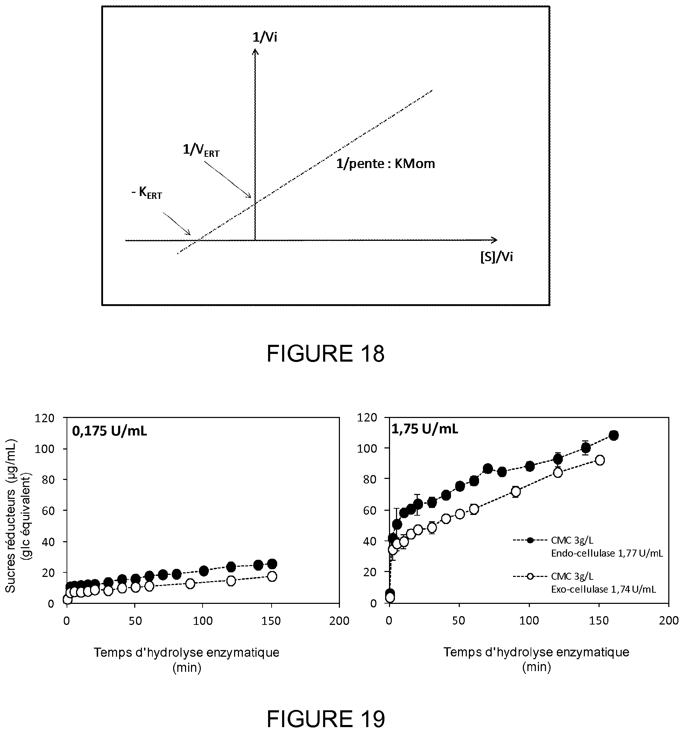

La figure 18 représente la courbe obtenue avec en ordonnée 1/vi et en abscisse [S]/Vi et la détermination graphique des constantes enzymatiques à savoir -$K_{ERT}$, 1/$V_{ERT}$ et $K_{MOM}$ obtenues.

La figure 19 représente des courbes représentant le suivi par dosage colorimétrique de l'hydrolyse enzymatique de la CMC (3 g/L) par l'endocellulase C1184 et l'exocellulase CBH1 à la même activité enzymatique (0,175 et 1,75 U/mL). L'ordonnée représente l'indice BFI et l'abscisse le temps en minutes. Sur la figure, les ronds pleins représentent la mesure lorsque le milieu comprend une concentration de CMC égale à 3g/L en présence d'endo-cellulase, les ronds vides représentent la mesure lorsque le milieu comprend une concentration de CMC égale à 3g/L en présence d'exo-cellulase.

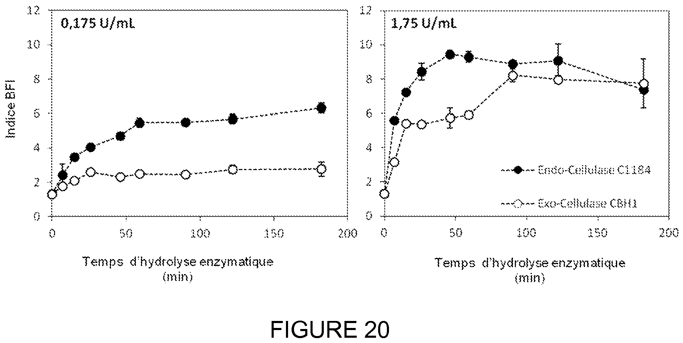

La figure 20 représente des courbes représentant le suivi de l'hydrolyse enzymatique de la CMC (3 g/L) par l'endocellulase C1184 et l'exocellulase CBH1 à la même activité enzymatique (0,175 et 1,75 U/mL). L'ordonnée représente l'indice BFI et l'abscisse le temps en minutes Sur la figure, les ronds pleins représentent la mesure lorsque le milieu comprend une concentration de CMC égale à 3g/L en présence d'endo-cellulase, les ronds vides représentent la mesure lorsque le milieu comprend une concentration de CMC égale à 3g/L en présence d'exo-cellulase.

**EXEMPLES**

**Exemple 1** : **Exemple de procédé de détermination de l'activité enzymatique et comparaison avec des procédés de l'état de la technique**

Dans cet exemple, un exemple de procédé selon l'invention a été mis en oeuvre afin de quantifier des activités enzymatiques dépolymérisantes de polysaccharides et la discriminer l'activité exo- et endo- dépolymérases. Deux polysaccharides, à savoir de la carboxyméthylcellulose ou de l'alginate et trois enzymes de clivage de ces polymères, à savoir une endocellulase, une exocellulase et une alginate lyase ont été utilisés.

Dans un premier temps, les constantes Michaëliennes (Km, Vmax) de ces trois enzymes ont été déterminées par des méthodes classiques d'enzymologie Michaëlienne à savoir courbe de Michaëlis et la méthode des doubles inverses de Lineweaver-Burk. Dans un deuxième temps, un exemple de procédé selon l'invention a rendu des résultats permettant d'accéder à des paramètres enzymatiques, nommés $V_{ERT}$, $K_{ERT}$ et $K_{MOM}$, comparables à des constantes Michaëliennes classiques. Les résultats obtenus par procédé selon l'invention sont cohérents avec ceux obtenus par des méthodes conventionnelles et ce procédé a permis de détecter des activité enzymatique et de discriminer des activités enzymatiques de type exo- et endo-.

**1.** Détermination des constantes Michaëliennes par colorimétrie et libération d'extrémités réductrices

Les réactions enzymatiques ont été menées en réacteur Carousel Tech 12 Plus Reaction Station (Radleys). Le substrat, à savoir la carboxyméthylcellulose (CMC) solubilisée sous agitation à 40°C, à des concentrations comprises entre 3 à 6 g/L ou l'alginate solubilisé sous agitation à température ambiante à des concentrations comprises entre 5 à 8 g/L a été indépendamment mis en contact avec une enzyme, à savoir l'endocellulase C1184 testée à $1,2.10^{-3}$, $1,2.10^{-2}$ et $1,2.10^{-1}$ U/mL, l'exocellulase CBH1 à $5.10^{-3}$ et $5.10^{-4}$ U/mL et l'alginate lyase E-ALGS à $1.10^{-3}$, $2.10^{-2}$ et $2.10^{-1}$ U/mL (U correspond à l'activité enzymatique définie par les différents fournisseurs, *i.e.* $\mu$mol.min$^{-1}$ pour le substrat concerné) (Tableau 1) ont été mis en contact à t=0min. Les couples enzymes substrats sont représentés dans le tableau 1 ci-dessous.

Tableau 1. Couples enzymes/substrats.

| Enzyme | pH | Substrat | Fournisseur |
|---|---|---|---|
| Cellulase (C1184) (EC 3.2.1.4) *Aspergillus niger* | 5,6 | Carboxyméthylcellulose (CMC) [21904, Fluka] | Sigma-Aldrich |
| CellobioHydrolase I (CBH1) (EC 3.2.1.91) *Trichoderma sp.* | 4,8 | Carboxyméthylcellulose (CMC) [21904, Fluka] | Megazyme |
| Alginate lyase (E-ALGS) (EC 4.2.2.3) *Sphingomonas sp.* | 7,2 | Sodium alginate [E401 RARE SEA, Cargill] | Megazyme |

La libération d'extrémités réductrices a été suivie de manière quantitative par la méthode développée par Waffenschmidt & Jaenicke (1987) à l'acide bicinchoninique (BCA) (Tableau 2) en spectrophotométrie (UV1700 PharmaSpec, Shimadzu) à une longueur d'ondes de $\lambda$=540 nm. Le tableau 2 ci-dessous présente les réactifs utilisés pour préparer la solution de travail pour le dosage BCA. La gamme étalon a été réalisée avec des concentrations ($\mu$g/mL) connues en glucose (49150, Fluka). Les réactions enzymatiques ont été réalisées sous agitation, à 20°C et au pH optimum de l'enzyme utilisée (Tableau 1). Au cours des cinétiques enzymatiques, chaque prélèvement subit un traitement thermique (20 min à 100°C) pour inactiver l'enzyme utilisée. Il a été vérifié que le traitement thermique ne dépolymérisait pas, ou de manière négligeable, les polymères utilisés. Les échantillons ont été stockés à -20°C avant leur analyse par dosage colorimétrique. Chaque cinétique enzymatique a été menée en triplicata, tout comme les dosages colorimétriques.

Tableau 2. Réactifs utilisés pour préparer la solution de travail (dosage BCA).

| Produit (N° catalogue) | Fournisseur |
|---|---|
| NaHCO$_3$ (123360010) | Acros Organic |
| Na$_2$CO$_3$ (207810010) | Acros Organic |
| Acide 2,2'-biquinoline-4,4'-dicarboxylique Sel trihydrate de dipotassium (391778) | Sigma-Aldrich |
| L-Sérine (S-8407) | Sigma-Aldrich |
| CuSO$_4$,5 H$_2$O (2790) | Merck |

1.1. Couple CMC/ENDOCELLULASE C1184

L'endocellulase choisie est connue pour avoir un comportement Michaëlien en milieu conventionnel. Les expérimentations ont été réalisées dans un tampon acétate de sodium 0,1 M à pH 5,6 (acétate de sodium trihydraté 6267, Merck ; acide acétique 33209, Sigma-Aldrich). Dans un premier temps, différentes concentrations/activités d'enzymes, à savoir C1 (1,2.10$^{-3}$ U/mL), C1/10 (1,2.10$^{-2}$ U/mL) et C1/100 (1,2.10$^{-1}$ U/mL) ont été testées en présence d'une solution tampon à 3 g/L de CMC. La figure 4 présente les résultats obtenus. Il a été observé logiquement que la concentration en enzymes la plus haute permet d'atteindre les concentrations en équivalent glucose les plus importantes. La concentration théorique annoncée par le fournisseur, i.e. 1,2.10$^{-2}$ U/mL, a été choisie pour accéder aux paramètres Michaëlien de l'enzyme. La figure 5, présente les résultats obtenus en faisant varier la concentration en CMC de 3 à 6 g/L en présence de cette concentration enzymatique. Des prélèvements réalisés durant les cinq premières minutes ont permis de déterminer précisément les valeurs des vitesses initiales pour chaque expérimentation (Tableau 3).

La courbe de saturation Vi = f ([S]) et la représentation graphique des doubles inverses (Lineweaver-Burk) ont ensuite été tracées conformément aux procédures classiques pour les enzymes Michaëliennes (Figure 6).

Le tableau 3 ci-dessous représente la détermination des vitesses initiales pour le couple CMC l'endocellulase C1184

Tableau 3. Détermination des vitesses initiales lors de l'hydrolyse enzymatique de la CMC (0 à 6 g/L) par l'endocellulase C1184 à 1,2.10-3 U/mL.

| [CMC] (g/L) | 1/[S] (L/g) | Vi ($\mu$mol/mL/min) | 1/Vi (mL.min/$\mu$mol) |
|---|---|---|---|
| 0 | - | 0 | - |
| 3 | 0,333 | 0,082 | 12,22 |
| 4 | 0,250 | 0,095 | 10,56 |
| 5 | 0,200 | 0,108 | 9,246 |
| 6 | 0,167 | 0,109 | 9,144 |

Ainsi, il a été possible d'accéder aux paramètres enzymatiques spécifiques de cette enzyme pour la CMC à savoir Vm = 0,177 U/mL et Km = 3,475 g/L.

La Vm obtenue est l'activité enzymatique dans le réacteur. En prenant en compte les dilutions réalisées au cours des expérimentations, l'activité enzymatique de la solution mère de départ a pu être calculée. Un dosage des protéines par la méthode au réactif de Folin (Lowry et al., 1951) a permis d'accéder à la concentration de protéines dans la solution mère d'enzymes et donc à l'activité spécifique AS réelle de l'endocellulase C1184 à savoir :A = 372 U/mL, [P]C1184 = 0,0567 g/L et AS = 657,2 U/mg.

1.2. Couple CMC/EXOCELLULASE CBH1

L'exocellulase choisie est connue pour avoir un comportement Michaëlien en milieu conventionnel. Les expérimentations ont été réalisées dans un tampon acétate de sodium 0,5 M à pH 4,8 (acétate de sodium trihydraté 6267, Merck ; acide acétique 33209, Sigma-Aldrich). Dans un premier temps, différentes concentrations/activités d'enzymes, à savoir C1 (5.10$^{-3}$ U/mL) et C1/10 (5.10$^{-4}$ U/mL) ont été testées en présence d'une solution tampon à 3 g/L de CMC. La même démarche expérimentale, décrite pour le couple CMC/endonucléase C1184, a permis d'accéder aux constantes Michaëliennes de l'enzyme. La concentration théorique annoncée par le fournisseur, i.e. 5.10$^{-4}$ U/mL, a été choisie pour accéder aux paramètres Km et Vm de l'enzyme. La figure 7, présente les résultats obtenus en faisant varier la concentration en CMC (3 à 6 g/L). Des prélèvements ont permis de déterminer les valeurs des vitesses initiales pour chaque expérimentation et sont représentées dans le tableau 4 ci-dessous.

Tableau 4. Détermination des vitesses initiales (pentes des tangentes à l'origine) lors de l'hydrolyse enzymatique de la CMC (0 à 6 g/L) par l'exocellulase CBH1 à $5.10^{-4}$ U/mL.

| [CMC] (g/L) | 1/[S] (L/g) | Vi ($\mu$mol/mL/min) | 1/Vi (mL.min/$\mu$mol) |
|---|---|---|---|
| 0 | - | 0 | - |
| 3 | 0,333 | 0,071 | 14,13 |
| 4 | 0,250 | 0,081 | 12,35 |
| 5 | 0,200 | 0,095 | 10,50 |
| 6 | 0,167 | 0,099 | 10,10 |

La courbe de saturation Vi = f ([S]) et la représentation graphique des doubles inverses (Lineweaver-Burk) ont ensuite été tracées conformément aux procédures classiques d'études enzymatiques avec comportement Michaëlien (Figure 8).

Ainsi, il a été possible d'accéder aux paramètres enzymatiques spécifiques de cette enzyme pour la CMC à savoir Vm= 0,174 U/mL et Km= 4,393 g/L

L'activité enzymatique de la solution mère de départ a été calculée comme précédemment. Un dosage des protéines par la méthode au réactif de Folin (Lowry et al., 1951) a permis d'accéder à la concentration de protéines dans la solution mère d'enzymes et donc à l'activité spécifique AS réelle de l'exocellulase CBH1 à savoir A = 174 U/mL, [P]C1184 = 11,3 g/L et AS = 15,4 U/mg.

1.3. Couple CMC/alginate lyase E-ALGS

L'alginate lyase choisie est connue pour avoir un comportement Michaëlien en milieu conventionnel. Les expérimentations ont été réalisées dans un tampon tris-HCl 0,1 M à pH 7,2 (Tris base 161-0719, Biorad ; acide chlorhydrique 30721, Riedem-de-Haën). Dans un premier temps, différentes concentrations d'enzymes, à savoir C1 ($2.10^{-1}$ U/mL), C1/10 ($2.10^{-2}$ U/mL) et C1/200 ($1.10^{-3}$ U/mL) ont été testées en présence d'une solution tampon à 5 g/L d'alginate. La même démarche expérimentale, décrite pour le couple CMC/endonucléase C1184, a permis d'accéder aux constantes Michaëliennes de l'enzyme. La concentration théorique annoncée par le fournisseur, i.e. $2.10^{-2}$ U/mL, a été choisie pour accéder aux paramètres Km et Vm de l'enzyme. La figure 9, présente les résultats obtenus en faisant varier la concentration en alginate (5 à 8 g/L). Des prélèvements supplémentaires réalisés durant les premières minutes ont permis de déterminer les valeurs des vitesses initiales pour chaque expérimentation et sont représentés dans le tableau 5 ci-dessous. La courbe de saturation Vi = f ([S]) et la représentation graphique des doubles inverses (Lineweaver-Burk) ont ensuite été tracées conformément aux procédures classiques d'études enzymatiques avec comportement Michaëlien (Figures 9 et 10). Ainsi, il a été possible d'accéder aux paramètres enzymatiques spécifiques de cette enzyme pour l'alginate :

Tableau 5. Détermination des vitesses initiales (pentes des tangentes à l'origine) lors de l'attaque enzymatique de l'alginate (0 à 7 g/L) par l'alginate lyase E-ALGS à $2.10^{-2}$ U/mL.

| [Alginate] (g/L) | 1/[S] (L/g) | Vi ($\mu$g/mL/min) | 1/Vi (mL.min/$\mu$g) |
|---|---|---|---|
| 0 | - | 0 | - |
| 5 | 0,2 | 0,119 | 8,41 |
| 6 | 0,167 | 0,143 | 7,00 |
| 7 | 0,143 | 0,148 | 6,75 |

**2.** Détermination des caractéristiques des enzymes avec un exemple de mise en oeuvre du procédé selon l'invention

Dans cette partie, les couples enzymes - substrats, tampons et conditions de mise en oeuvre sont identiques aux couples, tampons et conditions expérimentales mentionnés au point 1 ci-dessus.

En outre, pour chaque expérience a été introduit dans le contenant du Ton 004N à raison de 10$\mu$L/mL, du

tampon acétate de Na 100mM pH 5,6 et une endocellulase C1184, ou du Ton 004N à raison de 10$\mu$L/mL du tampon acétate de Na 50mM pH 4,8 et l'exocullase CBH1, ou du Ton 004N à raison de 10$\mu$L/mL, de l'alginate lyase E-ALGS, du tampon Tris-HCl 100mM pH 7,2. A un temps $t_0$ donné une première mesure du DL a été réalisée et la plaque apposée sur un bloc test comportant 96 aimants. A un second temps $t_{>0}$ donné une seconde mesure du DL a été réalisée et la plaque apposée sur un bloc test comportant 96 aimants.

Chaque cinétique enzymatique a été réalisée dans un volume de 2 mL, à concentration constante en enzymes, en faisant varier la concentration du substrat testé (CMC ou alginate) entre la concentration bloquante-1 (Cbloq-1g/L) et la concentration bloquante+3 (Cbloq+3 g/L). La concentration bloquante correspond à la concentration minimale à laquelle les billes magnétiques sont immobilisées dans la solution polysaccharidique de par sa viscosité ou de par la création d'un réseau entre les macromolécules. Un indice de dépolymérisation, nommé BioFilm Indice (BFI) ou Enzyme index EI, traduit directement la capacité des billes à se déplacer dans la solution sous l'influence d'un aimant au cours du temps suite à une dégradation des polymères par des enzymes (dépolymérisation du polysaccharide). On considère cette concentration bloquante atteinte lorsque l'indice BFI est inférieur à 2 (Badel et al., 2011). Pour chaque essai, 0,002% (w/v) de Tween ont été ajoutés ainsi que 10 $\mu$L/mL de Toner 004N.

Les figures 1 et 3 représentent des courbes représentant la valeur de l'indice BFI en fonction du pourcentage de billes mobiles dans le milieu.

Chaque série d'expérimentations comprend (prélèvement de 200 $\mu$L à chaque temps t) :

- un témoin tampon,
- un témoin tampon + enzymes,
- un témoin tampon + polysaccharide à différentes concentrations,
- les essais enzymatiques (tampon + polysaccharide + enzymes).

Les deux premiers témoins doivent présenter des valeurs de BFI constantes au cours du temps, comprises entre 12 et 14. Ils permettent de vérifier que le tampon seul ne retient pas le Toner et que l'ajout d'enzymes ne modifie pas cette caractéristique, i.e. ne piège pas les billes magnétiques (artefact). Les témoins substrats sans enzymes doivent présenter des valeurs de BFI constantes et inférieures à 2 (concentrations bloquantes). Le procédé selon l'invention a été utilisé sur les couples CMC/endocellulase C1184, CMC/exocellulase CBH1 et alginate/alginate lyase E-ALGS. Les mêmes paramètres de température (20°C) et pH décrits au point 1 ci-dessus (Tableau 1) ont été utilisés pour chaque couple.

2.1 Couple CMC/alginate lyase E-ALGS

La figure 11 présente les résultats obtenus concernant les témoins tampon, tampon + endocellulase C1184 et tampon + CMC à différentes concentrations. Comme représenté sur cette figure, les valeurs de BFI pour les différents témoins sont stables au cours du temps. La Cbloq-1g/L avec un indice BFI à 3,7 n'est pas suffisante pour immobiliser totalement les billes magnétiques. Cependant, une cinétique de libération des billes a été observable au cours des hydrolyses enzymatiques (Figure 11). La figure 11 présente les réactions enzymatiques du couple CMC/endocellulase C1184 suivies pendant 1h. Plus la concentration en CMC est importante, moins les valeurs des indices BFI sont élevées. Il s'agit d'un effet d'encombrement des sites de coupure lié à la viscosité importante (réseau polymérique dense) du milieu à fortes concentrations en substrats. Ce phénomène est lié à l'utilisation de microbilles paramagnétiques colorées soumises à un champ magnétique, électrique ou électromagnétique et utilisées comme marqueurs de variation de viscosités dans un milieu. Ceci empêche l'application de modèles Michaëliens classiques.

Les prélèvements réalisés durant les premières minutes ont permis de déterminer les valeurs des vitesses initiales (BFI/min) pour chaque expérimentation. De la même manière, des Vi ont été également calculées pour les concentrations intermédiaires en CMC 2,5, 3,5 ; 4,5 et 5,5 g/L (Tableau 6).

Tableau 6. Détermination des vitesses initiales (pente des tangentes à l'origine) lors de l'hydrolyse enzymatique de la CMC (2 à 6 g/L) par l'endocellulase C1184 (0,177 U/mL).

| [CMC] (g/L) | Vi (BFI/min) |
|---|---|
| 2 | 0,1449 |
| 2,5 | 0,1397 |
| 3,5 | 0,1187 |
| 4 | 0,0789 |
| 4,5 | 0,071 |

(suite)

| [CMC] (g/L) | Vi (BFI/min) |
|---|---|
| 5 | 0,0254 |
| 5,5 | 0,0191 |
| 6 | 0,0146 |

2.2 Couple CMC/EXOCELLULASE CBH1

La figure 13 présente les résultats obtenus concernant les témoins tampon, tampon + exocellulase CBH1, tampon + CMC à différentes concentrations. On constate que les valeurs de BFI pour les différents témoins sont stables au cours du temps. Le même effet que précédemment a été observé pour des concentrations croissantes en CMC.

Les prélèvements réalisés durant les premières minutes ont permis de déterminer les valeurs des vitesses initiales (BFI/min) pour chaque expérimentation. De la même manière, des Vi ont été également calculées pour des concentrations intermédiaires en CMC (Tableau 7).

Tableau 7. Détermination des vitesses initiales (pente des tangentes à l'origine) lors de l'hydrolyse enzymatique de la CMC (2 à 6 g/L) par l'exocellulase CBH1 (0,174 U/mL).

| [CMC] (g/L) | Vi (BFI/min) |
|---|---|
| 2 | 0,0727 |
| 3 | 0,024 |
| 3,5 | 0,0172 |
| 4 | 0,0105 |
| 4,5 | 0,0096 |
| 5 | 0,0062 |
| 5,5 | 0,0033 |
| 6 | 0,0015 |

2.3 Couple CMC/ENDOCELLULASE C1184

La figure 15 présente les résultats obtenus concernant les témoins tampon, tampon + alginate lyase E-ALGS, tampon + alginate à différentes concentrations. Il a été constaté que les valeurs de BFI pour les différents témoins sont stables au cours du temps. La figure 15 présente les réactions enzymatiques du couple alginate/alginate lyase E-ALGS suivies pendant 1h. Le même effet que précédemment a été observé pour des concentrations croissantes en alginate.

Les prélèvements réalisés durant les premières minutes ont permis de déterminer les valeurs des vitesses initiales (BFI/min) pour chaque expérimentation. De la même manière, des Vi ont été également calculées pour des concentrations intermédiaires en alginate (Tableau 8).

| [Alginate] (g/L) | Vi (BFI/min) |
|---|---|
| 4 | 0,2361 |
| 4,5 | 0,2199 |
| 5 | 0,1474 |
| 5,5 | 0,1017 |
| 6 | 0,0657 |
| 6,5 | 0,0457 |
| 7 | 0,0236 |
| 7,5 | 0,0125 |

(suite)

| [Alginate] (g/L) | Vi (BFI/min) |
|---|---|
| 8 | 0,007 |

3. Interprétations des résultats obtenus avec un exemple de mise en oeuvre du procédé de l'invention

Le modèle Vi/[S] et la double inverse 1/Vi = f([S]/Vi) ont donné des résultats exploitables où :

$$\text{Equation 1 :} \quad 1/V\_i = a \times ([S])/V\_i + b$$

où a est la pente de la régression linéaire (R$^2$ > 0,99) et b l'ordonnée à l'origine de cette régression, a et b pouvant également être définis par les équations suivantes 2 et 3, déduites de l'équation 1.

$$\text{Equation 2 :} \quad b = (1 - a[S])/V\_i$$

$$\text{Equation 3 :} \quad a = (1 - bV\_i)/([S])$$

La figure 16 présente les résultats obtenus pour les différents couples en utilisant ce modèle. A partir de ces résultats, trois constantes cinétiques ont été déterminées et comparées aux résultats obtenus par des techniques classiques d'enzymologie :

$V_{ERT}$ comparable à une vitesse maximale pouvant être atteinte dans une réaction enzymatique,
$K_{ERT}$ comparable à une affinité pour le substrat,
$K_{MOM}$ comparable à une constante de mobilité minimum traduisant une concentration du substrat à partir de laquelle les billes sont totalement immobilisées

En particulier, $V_{ERT}$ (BFI/min) est définie comme la vitesse maximale qui peut être atteinte pour le couple substrat/enzyme.
$K_{ERT}$ (g.min.L$^{-1}$.BFI$^{-1}$) définit la capacité de l'enzyme à diminuer l'indice BFI. Il traduit d'une affinité pour le substrat. Plus $K_{ERT}$ est petit, plus la capacité de dégradation de l'enzyme pour le substrat est grande.
$K_{MOM}$ ou constante de mobilité minimale (g/L) est la concentration en substrat à partir de laquelle la vitesse d'évolution du BFI est nulle, i.e. billes magnétiques sont totalement immobiles.
Les valeurs de $V_{ERT}$, $K_{ERT}$, $K_{MOM}$ obtenues pour chacune des expériences ci-dessus sont résumées dans le tableau 9 ci-dessous.

Tableau 9. valeurs de $V_{ERT}$, $K_{ERT}$, $K_{MOM}$ obtenues des trois couples substrats/enzymes étudiés.

| Substrat/enzymes | Concentration bloquante (g/L) | $V_{ERT}$ (BFI/min) | $K_{ERT}$ (g.min.L$^{-1}$.BFI$^{-1}$) | $K_{MOM}$ (g/L) |
|---|---|---|---|---|
| CMC/endocellulase C1184 | 3 | 0,22 | 29 | 6,24 |
| CMC/exocellulase CBH1 | 3 | 0,040 | 156 | 6,19 |
| Alginate/alginate lyase | 5 | 0,29 | 28,1 | 8,10 |

L'ensemble des expérimentations menées préalablement et décrites dans les parties précédentes ont permis de comparer les cinétiques de dégradation enzymatique de la CMC par l'endocellulase C1184 et l'exocellulase CBH1, et ce par une technique classique de spectrophotométrie et par le procédé de l'invention. Pour ce faire, les réactions ont été menées dans des conditions expérimentales identiques, i.e. mêmes concentrations en substrat (g/L) et enzymes (U/mL). La figure 19 présente les résultats obtenus par la méthode classique. Il est observé logiquement une dégradation enzymatique plus rapide de la CMC par l'endocellulase C1184 due à son mécanisme hydrolytique. Cette observation est moins significative à des concentrations plus hautes en enzymes (1,75 U/mL). Les vitesses initiales mesurées (μg/mL/min) dans chaque condition sont très proches et ne permettent aucune discrimination significative entre activités endo- et exo-dépolymérisantes.

Les mêmes réactions ont été réalisées et suivies avec le procédé de l'invention. La figure 20 présente les résultats obtenus où il est clairement observé des différences entre les cinétiques de dégradation de la CMC par l'endocellulase C1184 et l'exocellulase CBH1 (d'autant plus visibles à faibles concentrations). Les vitesses initiales mesurées dans ces conditions sont significativement différentes tel que montré dans le tableau 10 ci-dessous.

Tableau 10. Vitesses initiales approximatives mesurées à l'aide des résultats obtenus dans les figures 19 et 20.

| Enzymes | Par dosage colorimétrique ($\mu$g/mL/min) | | Procédé selon l'invention (BFI/min) | |
|---|---|---|---|---|
| | 0,175 U/mL | 1,75 U/mL | 0,175 U/mL | 1,75 U/mL |
| Endocellulase C1184 | 3,91 | 17,8 | 0,156 | 0,612 |
| Exocellulase CBH1 | 2,18 | 15,4 | 0,064 | 0,264 |

Le tableau 11 ci-dessous comprend les résultats de $V_{ERT}$, $K_{ERT}$, $K_{MOM}$, Vm et Km obtenus

Tableau 11. Récapitulatif des paramètres enzymatiques mesurés par dosage colorimétrique et par le procédé de l'invention pour les couples CMC/endocellulase C1184, CMC/exocellulase CBH1 et alginate/alginate lyase E-ALGS.

| Enzymes | Vm (U/mL) | Km (g/L) | $V_{ERT}$ (BFI/mL) | $K_{ERT}$ (g.min.L$^{-1}$.BFI$^{-1}$) | $K_{MOM}$ (g/L) |
|---|---|---|---|---|---|
| Endocellulase C1184 | 0,18 | 3,48 | 0,22 | 29 | 6,24 |
| Exocellulase CBH1 | 0,18 | 4,40 | 0,04 | 156 | 6,19 |
| Alginate lyase E-ALGS | 0,43 | 12,9 | 0,29 | 28,1 | 8,10 |

Comme démontré dans cet exemple, les procédés connus de l'état de la technique ne permettent pas de différencier les Vm de l'endocellulase C1184 et l'exocellulase CBH1. En d'autres termes, les procédés de l'état de la technique ne permettent pas, de par l'utilisation, par exemple de procédés colorimétriques, de déterminer l'activité enzymatique.

Comme démontré dans cet exemple, le procédé selon l'invention, de part, notamment l'utilisation de microbilles, permet avantageusement de différencier distinctement les vitesses hydrolytiques grâce, notamment au paramètre $V_{ERT}$. En outre, l'obtention du paramètre cinétique $K_{ERT}$ permet également avantageusement de discriminer l'activité exo-d'une activité endo-d'un facteur supérieur à 5, à l'inverse de méthodes classiques.

Par ailleurs, la corrélation entre le BFI et le degré de liberté des particules a été étudiée. Pour l'évolution de la BFI en fonction de la quantité de billes immobilisées les billes ont été introduites dans le milieu à une concentration comprise de 0 à 10$\mu$L/mL pour mimer le 100 % immobilisation (0$\mu$L/mL) et l'entière mobilité (10$\mu$L/mL). Une corrélation entre le BFI et la variation du degré de liberté $\Delta$DL a été établie via l'élaboration d'une courbe dont la pente est égale à 1 tel que représenté sur la figure 3.

Tel que démontré dans la figure, le pourcentage de particules mobiles est directement corrélé à la valeur de l'index Biofilm. Aussi, tel que démontré dans cet exemple, le procédé de l'invention permet à la fois de déterminer l'activité enzymatique, de déterminer si une enzyme est endo ou exo, via notamment la mesure du degré de liberté de particules dans un milieu et/ou de l'index Biofilm ou index Enzyme.

**Exemple 2 : Exemple de dispositifs pour mettre en oeuvre le procédé de l'invention**

Le dispositif utilisé comprenant notamment une microplaque 96 puits, notamment des barrettes de puits à fond plat (Référence : MSW002B, BioFilm Control, France), des blocs tests aimantés (BKT-MSW002 BioFilm Control, France), une solution de microbilles paramagnétiques (Ton004N, Biofilm Control, France), un lecteur de microplaque, à savoir un scanner de documents (perfection V-750 PRO, Epson, USA) avec lequel une prise d'image a été effectuée avec le logiciel EpsonScan (Epson, USA), un ordinateur comprenant un système d'exploitation et le logiciel BFC Elements EZ (marque déposée) permettant notamment de déterminer la valeur EI à partir de la comparaison d'images.

**Listes** des références

1. Badel, S., Laroche, C., Gardarin, C., Petit, E., Bernardi, T., Michaud, P. A new method to screen polysaccharide cleavage enzymes. Enzyme and Microbial Technology 2011, 48: 248-252.
2. Lowry, O.H., Rosebrough, N.J., Lewis Farr, A., Randall, R.J. Protein measurement with the folin phenol reagent. The Journal of Biological Chemistry 1951, 193: 265-275.
3. Waffenschmidt, S., Jaenicke, L. Assay of reducing sugars in the nanomole range with 2,2'- bicinchoninate. Analytical Biochemistry 1987, 165: 337-340.
4. Chavant P, Gaillard-Martinie B, Talon R, Hébraud M, Bernardi T., A new device for rapid evaluation of biofilm formation potential by bacteria. J Microbiol Methods. 2007 Mar;68(3):605-12. Epub 2007 Jan 9.

**Revendications**

1. Procédé de détermination de l'activité enzymatique endo ou exo d'une enzyme test comprenant les étapes suivantes:

     a) Introduire dans un milieu liquide au moins une particule magnétique ou magnétisable ou chargée électriquement, ladite particule flottant à la surface du milieu liquide ou en suspension dans le milieu liquide ou reposant sur une surface immergée dans le milieu liquide;
     b) Introduire dans ledit milieu un substrat de ladite enzyme test à une concentration déterminée [S];
     c) Introduire dans ledit milieu comprenant ledit substrat ladite enzyme test ;
     d) Mesurer un premier degré de liberté (DL) de mouvement de ladite au moins une particule dans ledit milieu à un temps to correspondant à l'introduction de ladite enzyme dans ledit milieu ;
     e) Appliquer un champ magnétique ou électrique ou électromagnétique capable de mettre en mouvement ladite au moins une particule flottant à la surface du milieu de culture ou en suspension dans le milieu liquide ou reposant sur une surface immergée dans le milieu de culture pendant un temps déterminé;
     f) Mesurer au moins un second degré de liberté (DL) de mouvement de ladite au moins une particule dans ledit milieu à un second temps $t_{>0}$
     g) Calculer la variation de degré de liberté (DL), $\Delta$DL entre le second temps $t_{>0}$ par rapport au temps to ;
     h) Répéter les étapes a) à g) avec différentes concentrations dudit substrat ;
     i) A partir d'un graphique obtenu avec les valeurs mesurées faisant apparaitre en abscisses les valeurs de temps et en ordonnée les valeurs de $\Delta$DL, déterminer la vitesse enzymatique initiale ($v_i$) en $\Delta$DL par unité de temps pour différentes concentrations en substrat, correspondant à la tangente à l'origine dudit graphique,
     j) Déterminer la constante $K_{ERT}$ correspondant à la capacité de l'enzyme à modifier le degré de liberté de mouvement de ladite particule dans ledit milieu, à partir d'une courbe obtenue avec les valeurs mesurées en faisant apparaitre en abscisse 1/vi et en ordonnée [S]/vi correspondant à la formule suivante :

$$\frac{1}{V_i} = a \times \frac{[S]}{V_i} + b$$

     dans laquelle a est la pente de la courbe obtenue et b est l'ordonnée à l'origine, la valeur de $K_{ERT}$ étant égale à la valeur absolue de l'intersection de la courbe avec l'abscisse,
     k) Comparer la valeur de la constante $K_{ERT}$ obtenue à l'étape i) avec une constante $K_{ERT}$ de référence obtenue à partir d'une enzyme de référence dont l'activité enzymatique est préalablement connue pour ledit substrat, ladite comparaison permettant de déterminer l'activité enzymatique endo ou exo de ladite enzyme.

2. Procédé selon la revendication 1, dans lequel les étapes a) à c) sont réalisées successivement ou simultanément.

3. Procédé selon la revendication 1 ou 2, dans lequel ladite, au moins une, particule est une particule chargée électriquement, magnétique ou magnétisable ou recouverte d'au moins une couche magnétique ou magnétisable.

4. Procédé selon l'une quelconque des revendications précédentes, dans lequel ladite, au moins une, particule est soumise à un champ électromagnétique, éventuellement appliqué par impulsion.

5. Procédé selon l'une quelconque des revendications précédentes, dans lequel ladite, au moins une, particule est soumise à une augmentation progressive du champ électromagnétique.

6. Procédé selon l'une quelconque des revendications précédentes, dans lequel ledit champ magnétique est généré par des moyens générateurs de champ en mouvement.

**7.** Procédé selon l'une quelconque des revendications précédentes, dans lequel ladite, au moins une, particule est éclairée au moyen d'une source lumineuse pour détecter son mouvement.

**8.** Procédé selon l'une quelconque des revendications précédentes, dans lequel ladite, au moins une particule, est génératrice d'un signal.

**9.** Procédé selon la revendication précédente, dans lequel ladite, au moins une, particule, est fluorescente ou phosphorescente ou radioactive ou chimioluminescente ou réfléchissante.

**10.** Procédé selon l'une quelconque des revendications 1 à 9, **caractérisé en ce que** ledit milieu est homogène ou non homogène.

**11.** Utilisation d'un dispositif pour la mise en oeuvre du procédé de détermination de l'activité enzymatique d'une enzyme selon l'une quelconque des revendications 1 à 10.

**Patentansprüche**

**1.** Verfahren zur Bestimmung der enzymatischen Endo- oder Exoaktivität eines Testenzyms, das die folgenden Schritte umfasst:

a) Einbringen mindestens eines magnetischen oder magnetisierbaren oder elektrisch geladenen Partikels in ein flüssiges Medium, wobei das Partikel auf der Oberfläche des flüssigen Mediums schwimmt oder in Suspension in dem flüssigen Medium schwebt oder auf einer Oberfläche, eingetaucht in das flüssige Medium, ruht;
b) Einbringen eines Substrats des Testenzyms in einer festgelegten Konzentration [S] in das Medium;
c) Einbringen des Testenzyms in das Medium, das das Substrat umfasst;
d) Messen eines ersten Freiheitsgrads (FG) der Bewegung des mindestens einen Partikels in dem Medium zu einem Zeitpunkt $t_0$, der dem Einbringen des Enzyms in das Medium entspricht;
e) Anlegen eines magnetischen oder elektrischen oder elektromagnetischen Felds, das das mindestens eine auf der Oberfläche des Kulturmediums schwimmende oder in Suspension in dem flüssigen Medium schwebende oder auf einer Oberfläche, eingetaucht in das Kulturmedium, ruhende Partikel bewegen kann, während einer festgelegten Zeitdauer;
f) Messen mindestens eines zweiten Freiheitsgrads (FG) der Bewegung des mindestens einen Partikels in dem Medium zu einem zweiten Zeitpunkt $t_{>0}$
g) Berechnen der Veränderung des Freiheitsgrads (FG), ΔFG, zwischen dem zweiten Zeitpunkt $t_{>0}$ und dem Zeitpunkt $t_0$;
h) Wiederholen der Schritte a) bis g) mit verschiedenen Konzentrationen des Substrats;
i) Bestimmen der enzymatischen Anfangsgeschwindigkeit ($v_i$) in ΔFG pro Zeiteinheit für verschiedene Konzentrationen an Substrat aus einem Diagramm, das mit den gemessenen Werten erhalten wird, wobei auf der Abszisse jeweils die Werte für die Zeit und auf der Ordinate die Werte von ΔFG darstellt werden, was der Tangente durch den Ursprung des Diagramms entspricht,
j) Bestimmen der Konstanten $K_{ERT}$, die der Fähigkeit des Enzyms entspricht, den Freiheitsgrad der Bewegung des Partikels in dem Medium zu verändern, ausgehend von einer Kurve, die mit den Messwerten erhalten wird, indem man auf der Abszisse $1/v_i$ und auf der Ordinate $[S]/v_i$ aufträgt, entsprechend der folgenden Formel:

$$\frac{1}{V_i} = a \times \frac{[S]}{V_i} + b$$

,

wobei a die Steigung der erhaltenen Kurve ist und b der Schnittpunkt mit der Ordinate ist, wobei der Wert $K_{ERT}$ gleich dem Absolutwert des Schnittpunkts der Kurve mit der Abszisse ist,
k) Vergleichen des in Schritt i) erhaltenen Werts für die Konstante $K_{ERT}$ mit einer Referenzkonstanten $K_{ERT}$, die mit einem Referenzenzym erhalten wurde, dessen enzymatische Aktivität für das Substrat bereits bekannt ist, wobei der Vergleich die Bestimmung der enzymatischen Endo- oder Exoaktivität des Enzyms gestattet.

**2.** Verfahren nach Anspruch 1, wobei die Schritte a) bis c) aufeinanderfolgend oder gleichzeitig durchgeführt werden.

**3.** Verfahren nach Anspruch 1 oder 2, wobei das mindestens eine Partikel ein elektrisch geladenes, magnetisches oder magnetisierbares oder mit mindestens einer magnetischen oder magnetisierbaren Schicht überzogenes Partikel ist.

**4.** Verfahren nach einem der vorhergehenden Ansprüche, wobei das mindestens eine Partikel einem elektromagnetischen Feld ausgesetzt wird, das gegebenenfalls mittels Impuls angelegt wird.

**5.** Verfahren nach einem der vorhergehenden Ansprüche, wobei das mindestens eine Partikel einer allmählichen Erhöhung des elektromagnetischen Felds ausgesetzt wird.

**6.** Verfahren nach einem der vorhergehenden Ansprüche, wobei das Magnetfeld durch Mittel zur Erzeugung eines sich bewegenden Magnetfelds erzeugt wird.

**7.** Verfahren nach einem der vorhergehenden Ansprüche, wobei das mindestens eine Partikel mithilfe einer Lichtquelle beleuchtet wird, um seine Bewegung zu erfassen.

**8.** Verfahren nach einem der vorhergehenden Ansprüche, wobei das mindestens eine Partikel ein Signal erzeugt.

**9.** Verfahren nach dem vorhergehenden Anspruch, wobei das mindestens eine Partikel fluoreszierend oder phosphoreszierend oder radioaktiv oder chemolumineszierend oder reflektierend ist.

**10.** Verfahren nach einem der Ansprüche 1 bis 9, **dadurch gekennzeichnet, dass** das Medium homogen oder inhomogen ist.

**11.** Verwendung einer Vorrichtung für die Durchführung des Verfahrens zur Bestimmung der enzymatischen Aktivität eines Enzyms, nach einem der Ansprüche 1 bis 10.

**Claims**

**1.** A method for determining the endo or exo enzymatic activity of a test enzyme comprising the following steps:

  a. Introducing into a liquid medium at least one magnetic or magnetizable or electrically charged particle, said particle floating on the surface of the liquid medium or in suspension in the liquid medium or resting onto an immersed surface in the liquid medium;
  b. Introducing into said medium a substrate of said test enzyme at a determined concentration [S];
  c. Introducing into said medium comprising said substrate said test enzyme;
  d. Measuring a first degree of freedom (DL) of movement of said at least one particle in said medium at a time to corresponding to the introduction of said enzyme into said medium;
  e. Applying a magnetic or electric or electromagnetic field capable of moving said at least one particle floating on the surface of the culture medium or in suspension in the liquid medium or resting onto an immersed surface in the culture medium for a predetermined time;
  f. Measuring at least a second degree of freedom (DL) of movement of said at least one particle in said medium to a second time $t_{>0}$
  g. Calculating the variation of degree of freedom (DL), $\Delta$DL between the second time $t_{>0}$ with respect to time to;
  h. Repeating steps a) to g) with different concentrations of said substrate;
  i. From a graph obtained with the measured values showing on the abscissa the time values and on the ordinate the values of $\Delta$DL, determining the initial enzymatic speed ($v_i$) in $\Delta$DL per time unit for different substrate concentrations, corresponding to the tangent to the origin of said graph,
  j. Determine the $K_{ERT}$ constant corresponding to the ability of the enzyme to modify the degree of freedom of movement of said particle in said medium, from a curve obtained with the measured values by making appear on abscissa 1/vi and on ordinate [S]/vi corresponding to the following formula:

$$\frac{1}{V_i} = a \times \frac{[S]}{V_i} + b$$

where a is the slope of the curve obtained and b is the ordinate at the origin, the $K_{ERT}$ value being equal to the absolute value of the intersection of the curve with the abscissa,

k. Comparing the value of the constant $K_{ERT}$ obtained in step i) with a reference constant $K_{ERT}$ obtained from a reference enzyme whose enzymatic activity is previously known for said substrate, said comparison making it possible to determine the endo or exo enzymatic activity of said enzyme.

2. The method according to claim 1, wherein steps a) to c) are performed successively or simultaneously.

3. The method according to any one of claims 1 to 2, wherein said at least one particle is an electrically charged, magnetic, or magnetizable particle or coated with at least one magnetic or magnetizable layer.

4. Method according to anyone of the preceding claims, wherein said at least one particle is subjected to an electromagnetic field, optionally applied by pulse.

5. A method according to any one of the preceding claims, wherein said at least one particle is subjected to a gradual increase in the electromagnetic field.

6. The method according to any of the preceding claims, wherein said magnetic field is generated by moving field generating means.

7. The method according to any one of the preceding claims, wherein said at least one particle is illuminated by means of a light source to detect its motion.

8. A method according to any one of the preceding claims, wherein said at least one particle is generating a signal.

9. Method according to the preceding claim, wherein said at least one particle is fluorescent or phosphorescent or radioactive or chemiluminescent or reflective.

10. Method according to any one of claims 1 to 9, **characterized in that** said medium is homogeneous or non-homogeneous.

11. Use of a device for carrying out the method for determining the enzymatic activity of an enzyme according to any one of claims 1 to 10

FIGURE 1

FIGURE 2

FIGURE 3

FIGURE 4

FIGURE 5

FIGURE 6

FIGURE 7

FIGURE 8

FIGURE 9

FIGURE 10

FIGURE 11

FIGURE 12

FIGURE 13

FIGURE 14

FIGURE 15

FIGURE 16

FIGURE 17

FIGURE 18

FIGURE 19

FIGURE 20

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **BADEL, S. ; LAROCHE, C. ; GARDARIN, C. ; PETIT, E. ; BERNARDI, T. ; MICHAUD, P.** A new method to screen polysaccharide cleavage enzymes. *Enzyme and Microbial Technology,* 2011, vol. 48, 248-252
- **LOWRY, O.H. ; ROSEBROUGH, N.J. ; LEWIS FARR, A. ; RANDALL, R.J.** Protein measurement with the folin phenol reagent. *The Journal of Biological Chemistry,* 1951, vol. 193, 265-275
- **WAFFENSCHMIDT, S. ; JAENICKE, L.** Assay of reducing sugars in the nanomole range with 2,2'- bicinchoninate. *Analytical Biochemistry,* 1987, vol. 165, 337-340
- **CHAVANT P ; GAILLARD-MARTINIE B ; TALON R ; HÉBRAUD M ; BERNARDI T.** A new device for rapid evaluation of biofilm formation potential by bacteria. *J Microbiol Methods.,* Mars 2007, vol. 68 (3), 605-12